# EUROPEAN PATENT SPECIFICATION

(11) **EP 1 562 205 B1**
(45) Date of publication and mention of the grant of the patent: **09.04.2008**
(21) Application number: 05100623.7
(22) Date of filing: 31.01.2005
(51) Int. Cl.: H01G 9/20

(54) **Method of manufacturing a dye-sensitized solar cell**
Herstellungsmethode für eine Farbstoffsolarzelle
Procédé de fabrication des cellules solaires sensibilisées par un colorant

(30) Priority: 03.02.2004 KR 2004006929
(43) Date of publication of application: 10.08.2005
(73) Proprietor: Samsung SDI Co., Ltd., Suwon-si Gyeonggi-do (KR)
(72) Inventor: Ahn, Kwang-Soon, Samsung SDI Co., Ltd., Yongin-City, Kyeonggi-Do (KR); Lee, Ji-Won, Samsung SDI Co., Ltd., Yongin-City, Kyeonggi-Do (KR); Choi, Jae-Man, Samsung SDI Co., Ltd., Yongin-City, Kyeonggi-Do (KR); Shin, Byong-Cheol, Samsung SDI Co., Ltd., Yongin-City, Kyeonggi-Do (KR); Lee, Wha-Sup, Samsung SDI Co., Ltd., Yongin-City, Kyeonggi-Do (KR); Park, Joung-Won, Samsung SDI Co., Ltd., Yongin-City, Kyeonggi-Do (KR)
(74) Representative: Hengelhaupt, Jürgen

(56) References cited:
- EP-A- 1 020 881
- EP-A- 1 075 005
- EP-A- 1 237 166
- WO-A-99/63599
- SUZUKI K ET AL: "APPLICATION OF CARBON NANOTUBES TO COUNTER ELECTRODES OF DYE-SENSITIZED SOLAR CELLS" CHEMISTRY LETTERS, NIPPON KAGAKUKAI, TOKYO, JP, vol. 32, no. 1, 2003, pages 28-29, XP008025168 ISSN: 0366-7022

## Description

### FIELD OF THE INVENTION

The present invention relates to a method of manufacturing a dye-sensitized solar cell, and in particular, to a dye-sensitized solar cell with an improved electrode structure to enhance its energy efficiency.

### BACKGROUND OF THE INVENTION

A dye-sensitized solar cell is a cell that converts solar energy into electric energy based on photosynthesis. The production of dye-sensitized solar cells generally involves relatively easy processing steps and low production cost compared to the production of conventional silicon solar cells. Dye-sensitized solar cells may be used in making windows for the outer walls of buildings, or in making glass houses. A prominent study of dye-sensitized solar cells was made by Michael Gratzel of Ecole Polytechnique Federale de Lausanne (EPFL, Switzerland) in 1991.

A dye-sensitized solar cell has a first electrode with a dye-absorbed metallic oxide film, and a second electrode which faces and is spaced apart from the first electrode.
EP 1 020 881 A2 discloses a dye-sensitized solar cell comprising an counter electrode made of metal leads covered with a transparent conductive layer thus forming convex and concave portions. However, these portions do not have a surface roughness in a suitable range to enable the electrode to scatter incident light.
Suzuki et al. (Chemistry Letters Vol. 32, No. 1 (2003) p. 28-29) disclose a surface of the counter electrode being made up by a nano-sized carbon material, such as single wall carbon nanotubes (SWCNTs), carbon filaments and nanohoms. The use of nano-sized carbon materials is described to enlarge the surface area and thus enhancing the electrochemical activity.
EP 1 237 166 A2 discloses a dye-sensitized solar cell having a photo electrode (comprising a semiconductor electrode layer and a transparent electrode layer) and an opposing counter electrode. The electrodes have sawtooth shape having a reflection pattern of the incident light that enhances the energy conversion efficiency.

However, dye-sensitized solar cells tend to have low photoelectric conversion efficiencies, which limit their practical use. In order to address this problem, the sunlight absorption of the solar cell or the dye absorption thereof should be increased.

For this purpose, increasing the electrode reflectivity, usage of the light scattering particles, or usage of the metallic oxide particles up to the nanometer level have been proposed. However, such techniques are limited in their ability to improve the photoelectric conversion efficiency of the solar cell.

### SUMMARY OF THE INVENTION

According to the present invention as claimed in claim 1, a dye-sensitized solar cell is manufactured which inhibits the recombination of electrons and holes while scattering the incident light, thereby enhancing the photoelectric conversion efficiency.

The dye-sensitized solar cell includes a light-transmissive first electrode, and a second electrode that is spaced apart from and faces the first electrode. A dye-absorbed porous layer is formed on the first electrode. Convexo-concave portions are formed on the surface of the second electrode that faces the first electrode, and an electrolyte is impregnated in the space between the first and the second electrodes. As used herein, the term "convexo-concave" means having convex and concave portions.

The surface of the second electrode facing the first electrode has a surface roughness with a root mean square (Rms) of between about 1nm and 10 µm enabling the surface to scatter incident light.

The convexo-concave portions formed at the surface of the second electrode facing the first electrode have a shape selected from the group consisting of a stepped shape, a needle-like shape, a mesh shape, a scratch shape, and a scar shape.

The dye contains a metal complex of Ru, and another metal selected from the group consisting of Ir, Al, Pt, Pd, Eu and Pb, and combinations thereof.

The porous layer contains metallic oxide particles with a mean particle diameter of about 100nm or less. The metallic oxide particles of the porous layer preferably have a mean particle diameter of between about 10 and 40nm. The porous layer may further contain conductive particles. The porous layer may still further contain light scattering particles. The light scattering particles are formed of the same material as the metallic oxide particles of the porous layer, and have a mean particle diameter of about 100nm or more.

The first electrode includes a first transparent substrate. Examples of the materials used for the first transparent substrate include materials selected from the group consisting of polyethylene terephthalate (PET), polyethylene naphthalate (PEN), polycarbonate (PC), polypropylene (PP), polyimide (PI), and triacetate cellulose (TAC). A first conductive film is located over the first transparent substrate. Examples of the materials used for the first conductive film include materials selected from the group consisting of indium tin oxide (ITO), fluorine tin oxide (FTO), ZnO-Ga₂O₃, ZnO-Al₂O₃ and SnO₂-Sb₂O₃.

The second electrode also includes a second transparent substrate. A second conductive film is located over the second transparent substrate. Examples of the materials used for the second transparent substrate include materials selected from the group consisting of polyethylene terephthalate (PET), polyethylene naphthalate (PEN), polycarbonate (PC), polypropylene (PP), polyimide (PI), and triacetate cellulose (TAC). According to one embodiment of the invention, the second conductive film is a two-layer conductive film with a first layer applied to the second transparent substrate and a second layer applied to the first layer. Examples of the materials used for the first layer of the conductive film located on the second transparent substrate include materials selected from the group consisting of indium tin oxide (ITO), fluorine tin oxide (FTO), ZnO-Ga₂O₃, ZnO-Al₂O₃, and SnO₂-Sb₂O₃.

Examples of the materials used for the second layer of the conductive film include Pt, Ru, Rh, Pd, Os, Ir, WO₃, TiO₂, and combinations thereof.

In a method of manufacturing a dye-sensitized solar cell with first and second electrodes, a porous layer is first formed on a surface of the first electrode, and a dye is then absorbed on the porous layer. Convexo-concave portions are formed at a surface of the second electrode. The first and the second electrodes are then arranged such that the surface of the second electrode with the convexo-concave portions faces the porous layer of the first electrode, an electrolyte is injected between the first and the second electrodes, and the cell is sealed.

In the step of forming the convexo-concave portions at a surface of the second electrode, the surface of the first layer of the second electrode is rubbed with an abrasive cloth, is mechanically etched, or is chemically etched. Convexo-concave portions with shapes including a stepped shape, a needle-like shape, a mesh shape, a scratch shape, and a scar shape are formed.

After forming the convexo-concave portions on the first conductive layer coated on a transparent substrate, the second conductive layer is then coated on the first conductive layer. For example, the convexo-concave portions can be formed on the first conductive layer through scratching, and Pt can be deposited onto the first conductive layer to form the second conductive layer.

### BRIEF DESCRIPTION OF THE DRAWINGS

The above and other advantages of the present invention will become more apparent by describing preferred embodiments thereof in detail with reference to the accompanying drawings in which:

Fig. 1 is a cross sectional view of a dye-sensitized solar cell prepared by a method according to an embodiment of the present invention;

Fig. 2 is a photograph of a surface of a second electrode of a dye-sensitized solar cell according to an embodiment of the present invention; and

Fig. 3 is a graph illustrating the voltage-current density characteristic of dye-sensitized solar cells according to the Example and the Comparative Example.

### DETAILED DESCRIPTION

The present invention will be described more fully hereinafter with reference to the accompanying drawings, in which preferred embodiments of the invention are shown.

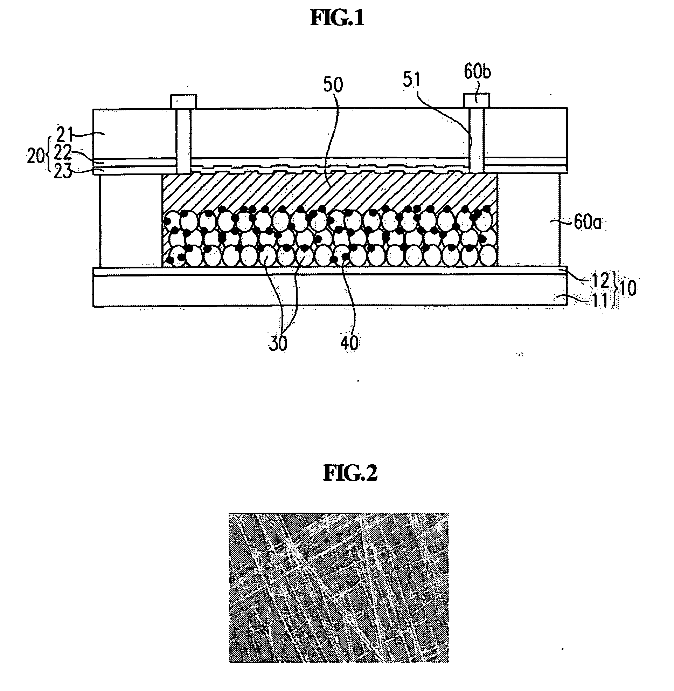

Fig. 1 is a cross sectional view of a dye-sensitized solar cell prepared by a method according to an embodiment of the present invention.

As shown in Fig. 1, the dye-sensitized solar cell includes a first electrode 10 and a second electrode 20 spaced apart from one another. A porous layer 30 is formed on the surface of the first electrode 10 facing the second electrode 20, and a dye 40 is absorbed on the porous layer 30. An electrolyte 50 is impregnated between the first and the second electrodes 10 and 20 to produce a dye-sensitized solar cell.

The first electrode 10 includes a transparent substrate 11, and a conductive film 12 coated on the substrate 11. Suitable materials for the substrate 11 include polyethylene terephthalate (PET), polyethylene naphthalate (PEN), polycarbonate (PC), polypropylene (PP), polyimide (PI), triacetate cellulose (TAC), and combinations thereof. The conductive film 12 is formed from a material such as indium tin oxide (ITO), fluorine tin oxide (FTO), ZnO-Ga₂O₃, ZnO-Al₂O₃, SnO₂-Sb₂O₃, and combinations thereof.

The porous layer 30 is formed on the surface of the first electrode 10 facing the second electrode 20. The porous layer 30 contains metallic oxide particles with a nanometer-leveled mean particle diameter. For example, the porous layer 30 may contain particles of TiO₂. The metallic oxide for the porous layer 30 preferably has a uniform particle diameter such that it can provide a high porosity and an optimal roughness.

In one embodiment of the invention, the metallic oxide particles of the porous layer 30 have a mean particle diameter of about 100nm or less, and preferably between about 10 and 40nm. When the mean particle diameter of the metallic oxide of the porous layer 30 is less than about 10nm, the adhesive force thereof is too weak to form the porous layer in a stable manner. When the mean particle diameter of the metallic oxide of the porous layer 30 exceeds about 40nm, the surface area of the dye-absorbed porous layer 30 is reduced so that the photoelectric conversion efficiency is decreased.

According to one embodiment of the invention, the porous layer 30 is formed by coating the inner surface of the first transparent substrate 11 with a paste, and heat-treating the coated first transparent substrate.

Various coating methods can be used, including use of a doctor blade or screen-printing techniques. In order to form the porous layer 30 as a transparent layer, spin coating or spraying may be used. Furthermore, it is also possible to use a common wet coating technique for that purpose. The physical properties of the paste may be modified depending upon the relevant coating technique used, and such modification would be apparent to one of ordinary skill in the art.

The paste may also include a binder. When a binder is added to the paste, the paste should be heat-treated at a temperature between about 450 and 600°C for about 30 minutes. Without a binder, it is possible to heat-treat the paste at a temperature of about 200°C or less.

The porous layer 30 may further contain a polymer to maintain the porosity thereof. In this case, the polymer is preferably selected from materials that leave little organic content after the heat treatment. Examples of such materials include polyethylene glycol (PEG), polyethylene oxide (PEO), polyvinyl alcohol (PVA), and polyvinyl pyrrolidone (PVP). In consideration of the coating conditions, a polymer with a proper molecular weight is selected, and added to the paste used to form porous layer 30. When the polymer is included in the porous layer 30, the porosity increases, and the diffusion and the viscosity of the porous layer 30 also increase, thereby enhancing the film formation and the adhesive force of the substrate.

The porous layer 30 may further contain conductive particles or light scattering particles. The conductive particles have a role in enabling the electrons to more easily migrate, and are formed with ITO. The light scattering particles have a role of lengthening the optical path and enhancing the photoelectric conversion efficiency, and are formed with the same material as the metallic oxide of the porous layer 30 but have a mean particle diameter of about 100nm or more.

A dye 40 is absorbed into the particle surface of the metallic oxide of the porous layer 30. The dye 40 is formed with a material capable of absorbing visible rays, such as a metal complex of Ru, and another metal selected from the group consisting of Ir, Al, Pt, Pd, Eu and Pb and combinations thereof. Ruthenium (Ru) is an element of the platinum group, and is capable of forming a number of organic metal complex compounds.

The dye material for the solar cell is most commonly selected from the type of Ru(etc bpy)₂(NCS)₂ 2CH₃CN. The "etc" is (COOEt)₂ or (COOH)₂ and is a radical capable of bonding with the surface of the porous layer 30 (for instance, the TiO₂). Furthermore, dyes for improving the absorption of visible light having long wavelengths to enhance the cell energy efficiency and new types of dyes capable of easily making the electron emission are under development and may be used.

The use of organic pigments such as coumarin, porphyrin, xanthene, riboflavin and triphenylmethane as dyes for a dye-sensitived solar cell has also been studied recently. Such organic pigments may be used alone or in combination with the Ru complex. Organic pigments are cost effective and abundant. Furthermore, the use of organic pigments makes it possible to improve the absorption of the long-wavelength visible rays, and enhance the cell energy efficiency.

In order to make the porous layer 30 absorb the dye 40, the first electrode 10 coated with the porous layer 30 is dipped in a solution of dye 40 dissolved in alcohol for about 12 hours.

The second electrode 20 facing the first electrode 10 has a transparent substrate 21 and a two-layer conductive film comprising a first conductive layer 22 coated on the substrate 21, and a second conductive layer 23 coated on the first conductive layer 22. The substrate 21 is formed from a material such as PET, PEN, PC, PP, PI and TAC and combinations thereof. The first conductive layer 22 is formed from a material such as ITO, FTO, ZnO-Ga₂O₃, ZnO-Al₂O₃, and SnO₂-Sb₂O₃ and combinations thereof. The second conductive layer 23 may contain a material such as Pt, Ru, Pd, Rh, Ir, Os, WO₃, TiO₂, C, and combinations thereof.

In order to form a second conductive layer 23 with Pt, a solution of H₂PtCl₆ dissolved in an organic solvent, for example, an alcohol such as methanol(MeOH), ethanol(EtOH) or isopropyl alcohol(IPA), is wet-coated onto the first conductive film 22 by way of spin coating, dip coating or flow coating, and is heat-treated at a temperature of about 400°C or more under air or oxygen atmosphere. Other possible coating methods include physical vapor deposition (PVD) techniques such as electrolyte plating, sputtering or electron beam deposition.

As noted above, convexo-concave portions may be formed on the surface of first conductive layer 22. As used herein, the term "convexo-concave" means having convex and concave portions. With the presence of the second conductive layer 23, the convexo-concave portions formed at the second conductive layer 23 correspond to those of the first conductive layer 22.

In consideration of the specific surface area, the effect of the light scattering and the practical processing, the root mean square (Rms) of the surface roughness of the film with the convexo-concave portions is preferably between about 1 nm and 10 µm. When the root mean square of the surface roughness is less than about 1nm, it is difficult to form the convexo-concave portions, and the light scattering effect deteriorates. When the root mean square of the surface roughness exceeds about 10 µm, the specific surface area is reduced too much.

The convexo-concave portions may have the shape of a stepped shape, a needle-like shape, a mesh shape, a scratch shape, and a scar shape. Methods of forming the convexo-concave portions on the first conductive layer 22 include rubbing with an abrasive cloth and mechanically etching or chemically etching the first conductive layer 22.

The electrolyte 50 is impregnated between the first and the second electrodes 10 and 20, and uniformly diffused to the inside of the porous layer 30. The electrolyte 50 is formed with a iodide/triiodide redox couple, and receives the electrons from the second electrode 20, and transfers them to the dye 40 through oxidation and reduction reactions. The voltage of the solar cell is determined by the energy level of the dye, and the difference in the level of oxidation and reduction of the electrolyte 50.

In a dye-sensitized solar cell according to the present invention, the first and second electrodes 10 and 20 are attached to each other using an adhesive 60a. Small holes 51 penetrate the second electrodes 20 to allow a solution for forming the electrolyte 50 to be injected therethrough and be impregnated into the space between the two electrodes 10 and 20. Once the electrolyte has been injected, any holes 51 are sealed using an adhesive 60b.

The adhesives 60a and 60b may be formed with a thermoplastic polymer film, such as SURLYN^{™}. The thermoplastic polymer film is disposed between the two electrodes, and thermally pressed. Epoxy resin or ultraviolet hardening agent may also be used for adhesives 60a and 60b, and if so, such material is hardened upon the heat treatment or UV treatment .

When sunlight is incident upon the solar cell, photons are first absorbed into the dye molecules, and the dye molecules are excited and electron-transferred from the ground state to the excited state to make electron-hole pairs. The electrons are transfered to the conduction band of the transition metal oxide forming the porous layer, and flow to the external circuit via the first electrode. Then, the electrons migrate to the second electrode. Meanwhile, the iodide I⁻ within the electrolyte is oxidized to triiodide I₃⁻, thereby reducing the oxidized dye. The triiodide I₃⁻ reacts with the electrons that reach the interface with the second electrode, and the triiodidide is reduced to iodide I⁻. In this way, the solar cell is operated by the migration of electrons.

In the dye-sensitized solar cell according to the present invention, the light incident through the first electrode is reflected or scattered by the convexo-concave portions of the second electrode 20. Thus, the optical path length within the solar cell is increased, and the reflected or scattered light again serves to generate additional electrons.

Furthermore, the convexo-concave portions at the second electrode 20 increase the contact area thereof with the electrolyte, and hence, the reduction capacity of the cell is increased. Consequently, the recombination of electrons with holes is inhibited, thereby increasing the open circuit voltage Voc and the short circuit current Isc. In the recombination of electrons the triiodide I³⁻ is recombined with the electrons generated by the dye, which reduces the number of electrons transferred to the conduction band of the metallic oxide of the porous layer.

Thus, in the dye-sensitized solar cell according to the present invention, the convexo-concave portions formed at the surface of the second electrode 20 serve to scatter the light and decrease electron recombination, thereby increasing the open circuit voltage and the short circuit current to provide enhanced energy efficiency.

A method of manufacturing a dye-sensitized solar cell will be further explained in accordance with an example of the present invention. The example is presented only to illustrate the present invention, which is not limited thereto.

### Example

First and second transparent substrates were prepared with ITO coated thereon as a conductive film. A TiO₂ paste with a mean particle diameter between about 7 and 25nm was bar-coated on about a 1 cm² section of the first substrate, and was fired at a temperature of about 450°C for about 30 minutes to form a porous layer with a thickness of about 15 µm.

Thereafter, about 0.3mM of Ru(4,4'-dicarboxy-2,2'-bipyridine)₂(NCS)₂ was dissolved in ethanol to form a solution, and the substrate with the porous layer was dipped in the solution at ambient temperature for about 12 hours to absorb the dye on the porous layer. The dye-absorbed porous layer was then cleaned, and dried at ambient temperature, thereby forming the first electrode.

The surface of the ITO coated on the second transparent substrate was then scratched, and then cleaned. Pt was deposited onto the ITO, using sputtering equipment, to a thickness of about 200nm, thereby forming the second electrode. In order to inject the electrolyte, a small hole was formed through the second electrode using a drill with a diameter of about 0.75mm. The surface roughness (Rms) of the second electrode measured after the scratching was about 300nm, and the surface resistance of the deposited Pt layer was about 2Ω /square.

Fig. 2 is a photograph of the surface of the second electrode not forming part of the present invention. As shown in Fig. 2, with the dye-sensitized solar cell according to the Example, the surface of the second electrode is covered with many scratches and is rough.

Next, the second electrode was positioned with its convexo-concave portions facing the porous layer of the first electrode, and a thermoplastic polymer film with a thickness of about 60 µm was placed between the first and the second electrodes and the construct was pressed together at a temperature of about 100°C for about 9 seconds, thereby attaching the electrodes to each other. An electrolyte solution was injected through the small hole formed in the second electrode, and thereafter the hole was sealed using a cover glass and a thermoplastic polymer film, thereby making the dye-sensitized solar cell. The electrolyte was made by dissolving about 21.928g of tetrapropylammonium iodide and about 1.931g of I₂ in a solvent of about 80% ethylene carbonate and about 20% acetonitrile.

### Comparative Example

A dye-sensitized solar cell was manufactured according to the Example except that the surface of the ITO of the second electrode was not scratched.

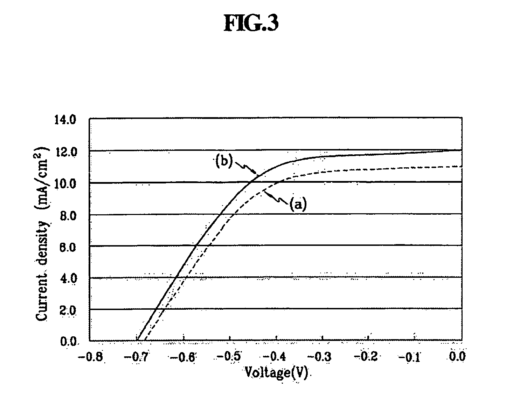

Fig. 3 is a graph illustrating the voltage-current density characteristic of the dye-sensitized solar cells according to the Comparative Example and the Example, wherein curve (a) illustrates the voltage-current density curve of the dye-sensitized solar cell according to the Comparative Example, and curve (b) illustrates the voltage-current curve of the dye-sensitized solar cell according to the Example.

The energy efficiency, the open circuit voltage, the short circuit current, and the fill factor (FF) of the dye-sensitized solar cells according to the Example and the Comparative Example were evaluated from the voltage-current density curves.

The dye-sensitized solar cell according to the Example exhibited about a 4.5% energy efficiency, about a 0.70V open circuit voltage, about a 11.84mA/cm² short circuit current, and about a 0.55 fill factor. The dye-sensitized solar cell according to the Comparative Example exhibited about a 4.05% energy efficiency, about a 0.67V open circuit voltage, about a 10.81mA/cm² short circuit current, and about a 0.55 fill factor.

The dye-sensitized solar cell according to the Example has an energy efficiency, an open circuit voltage and a short circuit current higher than that of the dye-sensitized solar cell according to the Comparative Example. This is because with the dye-sensitized solar cell according to the Example, electron recombination was inhibited by the convexo-concave portions formed on the surface of the second electrode, and because the dye was further excited by the light scattered due to the convexo-concave portions.

The reflectivity of the dye-sensitized solar cells according to the Example and the Comparative Example were measured with light having wavelengths between about 380 and 1100nm. The reflectivity of the dye-sensitized solar cell according to the Example was 27.4%, and the reflectivity of the dye-sensitized solar cell according to the Comparative Example was 53.1%. In the solar cell according to the Example, the light incident from the outside was reflected and scattered in the inside of the cell, thereby increasing the optical path length of the incident light.

Although preferred embodiments of the present invention have been described in detail hereinabove, it should be clearly understood that many variations and/or modifications of the basic inventive concept herein taught which may appear to those skilled in the art, as for as such embodiments still fall within the scope of the present invention, as defined in the appended claims.

## Claims

1. A method of manufacturing a dye-sensitized solar cell comprising:
preparing a first electrode (10) having a first surface and a second electrode (20) having a second surface;
forming a porous layer (30) on the first surface of the first electrode (10);
absorbing a dye (40) at the porous layer (30);
arranging the first and the second electrodes (10, 20) such that the second surface of the second electrode (20) faces the porous layer (30) of the first electrode (10); and
injecting an electrolyte (50) between the first electrode (10) and the second electrode (20),
**characterized in**
a step of forming convex and concave portions at the second surface of the second electrode (20) with a surface roughness with a root mean square (Rms) of between about 1 nm and 10 µm enabling the second surface to scatter incident light, the step comprising one of the methods of rubbing the surface of the second electrode (20) with an abrasive cloth, mechanically etching the surface thereof, and chemically etching the surface thereof.

2. The method of claim 1, wherein in the step of forming the convex and concave portions at a surface of the second electrode (20), the convex and concave portions are formed on a first conductive layer (22) coated on a transparent substrate (21), and a second conductive layer (23) is coated on the first conductive layer (22).

3. The method of claim 1, further comprising a step of sealing the solar cell after injecting the electrolyte (50).

4. The method of claim 1, wherein the dye (40) comprises a material capable of absorbing visible rays, selected from a metal complex of Ru or such as a metal complex of Ru and of another metal selected from the group consisting of Ir, Al, Pt, Pd, Eu and Pb, and combinations thereof.

5. The method of claim 1, wherein the porous layer 30) is formed of metallic oxide particles with a mean particle diameter of about 100 nm or less.

6. The method of claim 7, wherein the metallic oxide particles of the porous layer (30) have a mean particle diameter between about 10 and 40 nm.

7. The method of claim 5, wherein the porous layer (30) is further formed of conductive particles.

8. The method of claim 5, wherein the porous layer (30) is further formed of light scattering particles.

9. The method of claim 8, wherein the light scattering particles are comprised of the same material as the metallic oxide particles of the porous layer, and have a mean particle diameter of about 100nm or more.

10. The method of claim 1, wherein the first electrode (10) is prepared of a transparent substrate (11) comprising a polymer selected from the group consisting of polyethylene terephthalate (PET), polyethylene naphthalate (PEN), polycarbonate (PC), polypropylene (PP), polyimide (PI) and triacetate cellulose (TAC), and of a conductive film (12) coated on the substrate (11), the conductive film (12) comprising a compound selected from the group consisting of indium tin oxide (ITO), fluorine tin oxide (FTO), ZnO-Ga₂O₃, ZnO-Al₂O₃ and SnO₂-Sb₂O₃.

11. The method of claim 1, wherein the second electrode (20) is prepared of a transparent substrate (21) comprising a polymer selected from the group consisting of polyethylene terephthalate (PET), polyethylene naphthalate (PEN), polycarbonate (PC), polypropylene (PP), polyimide (PI) and triacetate cellulose (TAC), of a first conductive layer (22) coated on the substrate (21) comprising a compound selected from the group consisting of indium tin oxide (ITO), fluorine tin oxide (FTO), ZnO-Ga₂O₃, ZnO-Al₂O₃ and SnO₂-Sb₂O₃, and of a second conductive layer (23) coated on the first conductive layer (22).

12. The method of claim 11, wherein the second conductive layer (23) comprises an ingredient selected from the group consisting of Pt, Ru, Pd, Rh, Ir, Os, WO₃, TiO₂, and combinations thereof.

## Patentansprüche

1. Verfahren zur Herstellung einer Farbstoffsolarzelle, aufweisend:
Herstellung einer ersten Elektrode (10), die eine erste Oberfläche aufweist, und einer zweiten Elektrode (20), die eine zweite Oberfläche aufweist;
Ausbildung einer porösen Schicht (30) auf der ersten Oberfläche der ersten Elektrode (10);
Absorption eines Farbstoffs (40) an der porösen Schicht (30);
Anordnen der ersten und der zweiten Elektrode (10, 20) derart, dass die zweite Oberfläche der zweiten Elektrode (20) der porösen Schicht (30) der ersten Elektrode (10) zugewandt ist; und
Injektion eines Elektrolyten (50) zwischen der ersten Elektrode (10) und der zweiten Elektrode (20),
**gekennzeichnet durch**
einen Schritt zur Ausbildung konvexer und konkaver Abschnitte an der zweiten Oberfläche der zweiten Elektrode (20) mit einer Oberflächenrauheit, die einen quadratischen Mittelwert (Rms) zwischen etwa 1 nm und 10 µm aufweist und die zweite Oberfläche zur Streuung einfallenden Lichts befähigt,
wobei der Schritt eines der Verfahren des Schleifens der Oberfläche der zweiten Elektrode (20) mit einem Schleifleinen, des mechanischen Ätzens der Oberfläche der zweiten Elektrode (20) und des chemischen Ätzens der Oberfläche der zweiten Elektrode (20) aufweist.

2. Verfahren nach Anspruch 1, wobei im Rahmen des Schrittes der Ausbildung der konvexen und konkaven Abschnitte an einer Oberfläche der zweiten Elektrode (20) die konvexen und konkaven Abschnitte auf einer ersten leitfähigen Schicht (22), welche auf einem transparenten Substrat (21) aufgebracht ist, ausgebildet werden, und eine zweite leitfähige Schicht (23) auf der ersten leitfähigen Schicht (22) aufgebracht wird.

3. Verfahren nach Anspruch 1, weiterhin einen Schritt des Versiegelns der Solarzelle nach der Injektion des Elektrolyten (50) aufweisend.

4. Verfahren nach Anspruch 1. wobei der Farbstoff (40) ein Material, das sichtbare Strahlen absorbieren kann, aufweist, wobei das Material aus einem Metallkomplex von Ru ausgewählt ist oder beispielsweise ein Metallkomplex von Ru und von einem anderen Metall ist, welches aus der Gruppe bestehend aus Ir, Al, Pt, Pd, Eu und Pb und Kombinationen derselben ausgewählt ist.

5. Verfahren nach Anspruch 1, wobei die poröse Schicht (30) aus MetalloxidparcikeIn mit einem mittleren Partikeldurchmesser von etwa 100 nm oder weniger ausgebildet wird.

6. Verfahren nach Anspruch 7, wobei die Metalloxidpartikel der porösen Schicht (30) einen mittleren Partikeldurchmesser zwischen etwa 10 und 40 nm aufweisen.

7. Verfahren nach Anspruch 5, wobei die poröse Schicht (30) ferner aus leitfähigen Partikeln ausgebildet wird.

8. Verfahren nach Anspruch 5, wobei die poröse Schicht (30) ferner aus lichtstreuenden Partikeln ausgebildet wird.

9. Verfahren nach Anspruch 8, wobei die lichtstreuenden Partikel aus dem gleichen Material wie die Metalloxidpartikel der porösen Schicht bestehen und einen mittleren Partikeldurchmesser von etwa 100 nm oder mehr aufweisen.

10. Verfahren nach Anspruch 1, wobei die erste Elektrode (10) aus einem transparenten Substrat (11) hergestellt wird, welches ein Polymer aufweist, das aus der Gruppe bestehend aus Polyethylenterephthalat (PET), Polyethylennaphthalat (PEN), Polycarbonat (PC), Polypropylen (PP), Polyimid (PI) und Triacetat-Cellulose (TAC) ausgewählt ist, und aus einem leitfähigen Film (12), der auf dem Substrat (11) aufgebracht wird, wobei der leitfähige Film (12) eine Verbindung aufweist, die aus der Gruppe bestehend aus Indiumzinnoxid (ITO), Fluorzinnoxid (FTO), ZnO-Ga₂O₃, ZnO-Al₂O₃ und SnO₂-Sb₂O₃ ausgewählt ist.

11. Verfahren nach Anspruch 1, wobei die zweite Elektrode (20) aus einem transparenten Substrat (21) hergestellt wird, welches ein Polymer aufweist, das aus der Gruppe bestehend aus Polyethylenterephthalat (PET), Polyethylennaphthalat (PEN), Polycarbonat (PC), Polypropylen (PP), Polyimid (PI) und Triacetat-Cellulose (TAC) ausgewählt ist, und aus einer ersten leitfähigen Schicht (22), die auf dem Substrat (21) aufgebracht ist und die erste leitfähige Schicht (22) eine Verbindung aufweist, die aus der Gruppe bestehend aus Indiumzinnoxid (ITO), Fluorzinnoxid (FTO), ZnO-Ga₂O₃, ZnO-Al₂O₃ und SnO₂-Sb₂O₃ ausgewählt ist, und wobei die zweite Elektrode (20) weiterhin aus einer zweiten leitfähigen Schicht (23), die auf der ersten leitfähigen Schicht (22) aufgebracht ist, hergestellt wird.

12. Verfahren nach Anspruch 11, wobei die zweite leitfähige Schicht (23) einen Bestandteil aufweist, der aus der Gruppe bestehend aus Pt, Ru, Pd, Rh, Ir, Os, WO₃, TiO₂ und Kombinationen derselben ausgewählt ist.

## Revendications

1. Procédé de fabrication d'une cellule solaire sensibilisée par un colorant, comprenant:
la préparation d'une première électrode (10) ayant une première surface et d'une seconde électrode (20) ayant une seconde surface;
la formation d'une couche poreuse (30) sur la première surface de la première électrode (10);
l'absorption d'un colorant (40) à la couche poreuse (30);
l'agencement des première et seconde électrodes (10, 20) de façon que la seconde surface de la seconde électrode (20) soit tournée face à la couche poreuse (30) de la première électrode (10); et
l'injection d'un électrolyte (50) entre la première électrode (10) et la seconde électrode (20),
**caractérisé par**
une étape de formation de parties convexes et concaves à la seconde surface de la seconde électrode (20) avec une rugosité quadratique moyenne de surface (Rms) d'entre environ 1 nm et 10 µm permettant à la seconde surface de disperser une lumière incidente, l'étape comprenant l'un des processus de frottement de la surface de la seconde électrode (20) avec un tissu abrasif, d'attaque mécanique de sa surface, et d'attaque chimique de sa surface.

2. Procédé selon la revendication 1, dans lequel, dans l'étape de formation des parties convexes et concaves à une surface de la seconde électrode (20), les parties convexes et concaves sont formées sur une première couche conductrice (22) appliquée en revêtement sur un substrat transparent (21), et une seconde couche conductrice (23) est appliquée en revêtement sur la première couche conductrice (22).

3. Procédé selon la revendication 1, comprenant en outre une étape de scellement de la cellule solaire après l'injection de l'électrolyte (50).

4. Procédé selon la revendication 1, dans lequel le colorant (40) comprend une matière capable d'absorber des rayons visibles, choisie à partir d'un complexe métallique de Ru ou telle qu'un complexe métallique de Ru et d'un autre métal choisi dans le groupe constitué des Ir, Al, Pt, Pd, Eu et Pb, et des combinaisons de ceux-ci.

5. Procédé selon la revendication 1, dans lequel la couche poreuse (30) est formée de particules d'oxyde métallique ayant un diamètre moyen d'environ 100 nm ou moins.

6. Procédé selon la revendication 7, dans lequel les particules d'oxyde métallique de la couche poreuse (30) ont un diamètre moyen compris entre environ 10 et 40 nm.

7. Procédé selon la revendication 5, dans lequel la couche poreuse (30) est en outre formée de particules conductrices.

8. Procédé selon la revendication 5, dans lequel la couche poreuse (30) est en outre formée de particules dispersant la lumière.

9. Procédé selon la revendication 8, dans lequel les particules dispersant la lumière sont constituées de la même matière que les particules d'oxyde métallique de la couche poreuse, et ont un diamètre moyen d'environ 100 nm ou plus.

10. Procédé selon la revendication 1, dans lequel la première électrode (10) est préparée à partir d'un substrat transparent (11) comprenant un polymère choisi dans le groupe constitué de téréphtalate de polyéthylène (PET), de naphtalate de polyéthylène (PEN), de polycarbonate (PC), de polypropylène (PP), de polyimide (PI) et de triacétate-cellulose (TAC), et d'un film conducteur (12) appliqué en revêtement sur le substrat (11), le film conducteur (12) comprenant un composé choisi dans le groupe constitué d'oxyde d'étain et d'indium (ITO), d'oxyde d'étain et de fluor (FTO), de ZnO-Ga₂O₃, de ZnO-Al₂O₃ et de SnO₂-Sb₂O₃.

11. Procédé selon la revendication 1, dans lequel la seconde électrode (20) est préparée à partir d'un substrat transparent (21) comprenant un polymère choisi dans le groupe constitué de téréphtalate de polyéthylène (PET), de naphtalate de polyéthylène (PEN), de polycarbonate (PC), de polypropylène (PP), de polyimide (PI) et de triacétate-cellulose (TAC), d'une première couche conductrice (22) appliquée en revêtement sur le substrat (21) comprenant un composé choisi dans le groupe constitué d'oxyde d'étain et d'indium (ITO), d'oxyde d'étain et de fluor (FTO), de ZnO-Ga₂O₃, de ZnO-Al₂O₃ et de SnO₂-Sb₂O₃, et d'une seconde couche conductrice (23) appliquée en revêtement sur la première couche conductrice (22).

12. Procédé selon la revendication 11, dans laquelle la seconde couche conductrice (23) comprend un ingrédient choisi dans le groupe constitué de Pt, de Ru, de Pd, de Rh, d'Ir, d'Os, de WO₃, de TIO₂ et de combinaisons de ceux-ci.
